# EUROPÄISCHE PATENTSCHRIFT

(11) **EP 3 586 740 B1**
(45) Veröffentlichungstag und Bekanntmachung des Hinweises auf die Patenterteilung: **07.06.2023**
(21) Anmeldenummer: 19181263.5
(22) Anmeldetag: 19.06.2019
(51) Int. Cl.: A61B 5/08

(54) **SYSTEM UND VERFAHREN ZUR BEREITSTELLUNG VON ZUMINDEST EINER INFORMATION ÜBER ZUMINDEST EINE WECHSELWIRKUNG ZWISCHEN UMGEBUNGSLUFT UND ATEMFUNKTION VON EINEM INDIVIDUUM**
SYSTEM AND METHOD FOR PROVIDING AT LEAST ONE PIECE OF INFORMATION VIA AT LEAST ONE INTERACTION BETWEEN AMBIENT AIR AND RESPIRATORY FUNCTION OF AN INDIVIDUAL
SYSTÈME ET PROCÉDÉ DE FOURNITURE D'AU MOINS UNE INFORMATION SUR AU MOINS UNE INTERACTION ENTRE L'AIR AMBIANT ET LA FONCTION RESPIRATOIRE D'UN INDIVIDU

(30) Priorität: 21.06.2018 DE 102018114970
(43) Veröffentlichungstag der Anmeldung: 01.01.2020
(73) Patentinhaber: breazy-health GmbH, 14482 Potsdam (DE)
(72) Erfinder: Thom, Andreas, 13187 Berlin (DE); Mühlbauer, Felix, 14482 Potsdam (DE); Gnadt, Benedikt, 10967 Berlin (DE)
(74) Vertreter: Gulde & Partner

(56) Entgegenhaltungen:
- WO-A1-2014/076250
- WO-A1-2017/136336

## Beschreibung

Die Erfindung betrifft ein System zur Bereitstellung von zumindest einer Information über zumindest eine Wechselwirkung zwischen Umgebungsluft und Atemfunktion von einem Individuum sowie ein Verfahren zur Bereitstellung von zumindest einer Information über zumindest eine Wechselwirkung zwischen Umgebungsluft und Atemfunktion von einem Individuum.

Umwelteinflüsse haben zunehmend einen Einfluss auf die Gesundheit und das Wohlbefinden von Individuen. Insbesondere bei Menschen, welche in Ballungsräumen leben, können sich Umwelteinflüsse zu einer starken Belastung der Gesundheit entwickeln. Im Fokus stehen dabei unter anderem Umwelteinflüsse, welche direkt oder indirekt die Luftqualität betreffen. Dabei ist vor allem bei Personen mit Erkrankungen der unteren Atemwege ein unmittelbarer Zusammenhang zwischen Wohlbefinden und aktueller Exposition von Umwelteinflüssen festzustellen. Derartige Erkrankungen der unteren Atemwege können zum Beispiel Asthmaerkrankungen oder COPD (COPD = chronic obstructive pulmonary disease = chronisch obstruktive Lungenerkrankung) sein. Um diese Zusammenhänge besser verstehen zu können beziehungsweise um Informationen für einen entsprechenden Therapieansatz zu erhalten, sind aus dem Stand der Technik bereits erste Lösungsvorschläge offenbart worden.

So ist aus der US 6, 231, 519 B1 ein Verfahren und ein Gerät als offenbart zu entnehmen, mit welchem Risikofaktorinformationen und Standortdaten gesammelt werden, welche mit Asthmapatienten assoziiert sind. Die Informationen werden entsprechend für diese Patienten bereitgestellt, wobei insbesondere Informationen über die Luftqualität im Fokus stehen. Für die Übertragung der Informationen wird dabei ein klassisches Zwei-Wege-Kommunikationstool vorgeschlagen, um die Analyse der Luftqualität basierend auf menschlichen Reaktionen und Clustering-Methoden zu erleichtern. Das Verfahren umfasst das Messen vorbestimmter physiologischer und dynamischer Parameter, welche in Verbindung mit einem Benutzer stehen, wobei diese gemessenen Parameter anschließend an ein Analysewerkzeug übertragen werden. Nach der Analyse können dann Beratungsinformationen abgeleitet und dem Benutzer zur Verfügung gestellt werden. Das Messen vorbestimmter physiologischer und dynamischer Parameter, die mit einem Benutzer assoziiert sind, umfasst das Messen physiologischer Zustände des Benutzers, wie zum Beispiel Atemfunktionsanzeigen. Die dynamischen Parameter umfassen lokale Informationen wie die Tageszeit und/oder geografische Standorte. Das Senden wird unter Verwendung eines Mobiltelefons, eines mobilen 2-Wege-Pagers, eines drahtlosen persönlichen digitalen Assistenten oder einer Internet-Zugangsvorrichtung durchgeführt und das Analysieren umfasst das Abbilden der Daten unter Verwendung einer selbstorganisierenden Karte. Eine Wechselwirkung von Messparametern mit Bestandteilen der Atemluftströmungswege der Benutzer sowie eine Ableitung von beispielsweise Schadstoffraten beziehungsweise Mengen an reizenden Stoffen auf körperliche Bestandteile der Benutzer, wie zum Beispiel direkt der Lunge und der Atemluftströmungswege, wird dabei nicht vorgenommen.

Es wäre jedoch ein großer diagnostischer und pathophysiologischer Gewinn, wenn bei Personen mit oder ohne Erkrankungen der unteren Atemwege, zum Beispiel bei Personen mit Asthma oder COPD, analysiert werden könnte, wie viel feste Teilchen (korpuskularer Natur, zum Beispiel Feinstaub) oder gasförmige Anteile (zum Beispiel Stickstoffdioxyd oder Ozon) bei der Einatmung in der Lunge verbleiben. Zudem wäre es ein zusätzlicher Gewinn, wenn Umgebungsparameter in wechselnder Qualität, zum Beispiel innerhalb und außerhalb schadstoffbelasteter Stadtgebiete, in Verbindung zu Lungenfunktionsparametern aber auch zu Daten der gesundheitsbezogenen Lebensqualität (zum Beispiel SF 36 = Short Form 36 = Gesundheitsfragebogen) bei Individuen ohne oder mit obstruktiven Atemwegserkrankungen gesetzt werden können.

WO 2014/076250 A1 offenbart ein Inhalationsgerät für einen Patienten, bei dem eine gezielte Luftzusammensetzung erzeugt und dem Patienten zugeführt wird. Mittels eines Messgerätes kann die genaue Zusammensetzung der zu inhalierenden Luft bestimmt werden.

WO 2017/136336 A1 offenbart ein System, bei dem mittels Sensoren einzelne Parameter einer inhalierten und einer ausgeatmeten Luft eines Benutzers gemessen werden kann. Aus der zu inhalierenden Luft werden zuvor gesundheitsschädliche oder unangenehme Substanzen herausgefiltert.

Der Erfindung liegt nun die Aufgabe zugrunde, ein System und ein Verfahren bereitzustellen, mit welchen eine individuelle und genaue Erhebung von Informationen über zumindest eine Wechselwirkung zwischen Umgebungsluft und Atemfunktion von einem Individuum möglich ist. In bevorzugter Ausgestaltung der Erfindung ist vorgesehen, dass ein System zur Bereitstellung von zumindest einer Information über zumindest eine Wechselwirkung zwischen Umgebungsluft und Atemfunktion von einem Individuum bereitgestellt wird. Das System umfasst zumindest ein Peripheriegerät, zumindest eine Lungenfunktionsvorrichtung, zumindest eine mit der zumindest einen Lungenfunktionsvorrichtung gekoppelten Berechnungsvorrichtung umfassend zumindest eine Schnittstelle, sodass die Berechnungsvorrichtung ausgelegt ist, mit zumindest einem Peripheriegerät zumindest die Information auszutauschen, wobei das System zudem zumindest eine mit der zumindest einen Lungenfunktionsvorrichtung und/oder der zumindest einen Berechnungsvorrichtung gekoppelten Umweltsensoreinrichtung umfasst, welche ausgelegt ist, zumindest einen Messparameter zu messen und welche ausgelegt ist, diesen zumindest einen Messparameter sowohl bei zumindest einem Einatemvorgang des Individuums und bei zumindest einem Ausatemvorgang des Individuums zu messen, sodass mittels des jeweils zumindest einen gemessenen Messparameters beim Einatemvorgang und beim Ausatemvorgang zumindest die Information in Form einer Differenzmessung der gemessenen Messparameter ermittelt und an das System bereitgestellt wird.

Das System ist somit in der Lage, eine Information über die Wechselwirkung der Umgebungsluft und der damit einhergehenden Luftqualität und Beschaffenheit und der Atemfunktion eines Individuums abzuleiten. Ein Individuum könnte zum Beispiel ein Mensch sein. Aufgrund der Differenzmessung ist es also möglich, eine Aussage über den jeweiligen Messparameter zu treffen. Insbesondere kann eine Aussage beziehungsweise allgemein eine Information darüber bereitgestellt werden, inwiefern der jeweilige Messparameter eine Wechselwirkung mit dem Individuum eingegangen ist. Beispielsweise kann somit hergeleitet werden, welcher Anteil des gemessenen Messparameters bei einem Einatemvorgang beispielsweise in der Lunge verbleibt. Diese Information kann dem System bereitgestellt werden, sodass beispielsweise die Berechnungsvorrichtung im Zusammenhang mit der Lungenfunktionsvorrichtung entsprechende Informationen berechnen kann. Mit anderen Worten kann die Lungenfunktionsvorrichtung, welche beispielsweise ein Spirometer oder dergleichen sein kann, typischerweise Basisinformationen (Spirometrie, Peak-Flow oder FeNO - Messungen = Fraktioniertes exhaliertes Stickstoffmonoxid (NO)) bereitstellen, welche anschließend mit der Differenzmessung und den damit einhergehenden Informationsfluss an Daten verschnitten beziehungsweise kombiniert oder parallel jeweils einzeln einem Nutzer des Systems zur Verfügung gestellt werden kann. Ein Spirometer ist dazu ausgelegt, eine Messung der Atemfunktion generell durchzuführen.

Mit anderen Worten kann mittels der Lungenfunktionsvorrichtung optional eine Basismessung vollzogen werden, wobei die gemessenen Basisinformationen sinngemäß in dem System verwendet werden können. Somit ist mit dem Einbezug der Lungenfunktionsvorrichtung also implizit dieser Zusammenhang in dem vorgestellten System vorgesehen, ohne das hier eine weitere Ausführung von Grundlagen vorgenommen wird. Es kann also analysiert werden, welche Menge von dem Messparameter im Körper beispielsweise in der Lunge und/oder dem Atemluftströmungsweg verbleibt. Über die Schnittstelle können dann berechnete Informationen an ein oder mehrere Peripheriegeräte übermittelt beziehungsweise allgemein bereitgestellt werden. Andersrum können über das oder die Peripheriegeräte auch zusätzliche Informationen an die Berechnungsvorrichtung übermittelt beziehungsweise allgemein bereitgestellt werden. Auch kann beispielsweise ein Nutzer des Systems somit eine Systemeingabe tätigen beziehungsweise ausführen. Zum Beispiel können auf diese Weise Symptomeingaben getätigt werden, sodass das System diese bei seiner Funktionsweise entsprechend berücksichtigen kann. Das System kann so gestaltet sein, dass mehrere Messparameter parallel gemessen werden und somit eine zuverlässige Aussage über die Differenzmessung ermöglicht wird. Eine Berechnung kann dabei bereits in der Umweltsensoreinrichtung vorgenommen werden.

Denkbar sind jedoch auch, dass entweder eine Übermittlung an die Berechnungsvorrichtung vorgenommen wird und anschließend eine Berechnung stattfindet oder dass diese Berechnung jeweils nur anteilig durch die zuvor genannten Komponenten vollzogen wird. Auch ist es denkbar, dass anteilig eine Berechnung von der Lungenfunktionsvorrichtung vorgenommen wird und diese Information entsprechend der Umweltsensoreinrichtung zur Verfügung gestellt wird. Auch könnte eine Information von dem zumindest einen Peripheriegerät die Berechnung beispielsweise in der Umweltsensoreinrichtung veranlassen oder beeinflussen, sodass eine genaue und flexible Messung noch besser möglich ist. Die Berechnungsvorrichtung kann zudem ausgelegt sein, eventuelle Wechselwirkungen zwischen den jeweils für sich gemessenen Messparametern bei der Auswertung beziehungsweise Berechnung beziehungsweise schlicht bei der Feststellung der jeweiligen Differenzmessung zu berücksichtigen.

Die von einem beliebigen Peripheriegerät zugeführten Informationen können beispielsweise allgemeine Informationen über die Umgebungsluft sein, wobei die Datenquelle der Informationen entweder beliebig oder sogar vom Nutzer ausgewählt werden kann. Auch kann der Nutzer das System über die zumindest eine Schnittstelle individuell konfigurieren, je nach dem, welche Art der Nutzung beziehungsweise Aktivierung vorgesehen ist. So kann beispielsweise ein Asthmapatient eine individuelle Symptomeingabe festlegen oder etwa externe Informationen gefiltert nach jeweiligen Peripheriegeräten auswählen und dem System zur Verfügung stellen. Somit lassen sich beispielsweise Umgebungsparameter der Umgebungsluft in Bezug auf eine Luftqualität in einen jeweiligen Berechnungsvorgang einbinden.

Die Umweltsensoreinrichtung kann ein einfacher Sensor sein oder auch einen Zusammenschluss von verschiedenen Sensoren, welche jeweils auf ein Messparameter spezialisiert ausgebildet sind, umfassen. Auch kann die Umweltsensoreinrichtung über Mittel verfügen, welche es erlauben, eine voreingestellte oder individuelle, beispielsweise vom Nutzer gewählte, Verbindungsarten zu den anderen Komponenten des Systems herzustellen beziehungsweise bereitzustellen. Beispielsweise kann die Umweltsensoreinrichtung eine Funkverbindungseinrichtung umfassen, welche es erlaubt, sowohl Informationen zu empfangen als auch zu senden. So eine Funkverbindungseinrichtung kann beispielsweise die gemessene Differenzmessung an die Berechnungsvorrichtung senden, so dass diese Informationen entsprechend dem System für weitere Schritte zur Verfügung steht. Auch ist es vorstellbar, dass diese Informationen beziehungsweise Daten unmittelbar an ein externes Peripheriegerät gesendet werden. Beispielsweise kann somit ein Nutzer direkt diese Daten beziehungsweise Informationen über die in seinem Körper verbleibenden Stoffe beziehungsweise Mengen des jeweiligen Messparameters auslesen und anschließend individuelle Maßnahmen ergreifen.

Auch könnten diese Informationen von einer Vielzahl von Systemen parallel an ein oder mehrere externe Peripheriegeräte gesendet beziehungsweise bereitgestellt werden, wobei die Agglomeration derartiger Differenzmessungen dann bei einem externen Peripheriegerät bei Überschreitung von individuell festgelegten Ergebnissen beziehungsweise Grenzwerten eine oder mehrere Aktionen beziehungsweise Handlungsanweisungen ausgeben oder bereitgestellt werden können. Beispielsweise könnte dem Nutzer eines solchen Systems somit eine Rücckopplung in sein individuelles System eingespeist werden, sodass fortlaufende Messungen oder Berechnungsschritte beeinflusst oder allgemein gesteuert werden. Beispielsweise könnte ein Individuum durch die Lungenfunktionsvorrichtung ein- beziehungsweise ausatmen, wobei die Umweltsensoreinrichtung davor, danach oder anteilig davor und danach vorgesehen werden kann. Die Positionierung ist dabei stets so zu wählen, sodass die Funktionalitäten der einzelnen Komponenten gewährleistet sind.

So versteht es sich, dass eine eingeatmete Luftmenge mit den zu messenden Messparametern unbeeinflusst die Umweltsensoreinrichtung beziehungsweise den entsprechenden Sensor zu erreichen hat, sodass eine unverfälschte Messung gewährleistet wird. Die zu wählenden Materialien sollten diese Vorgabe entsprechend berücksichtigen. Demnach sind bei der Materialauswahl reaktionsträge Materialien zu bevorzugen, beziehungsweise generell Materialien, welche mit den zu messenden Messparametern nicht reagieren beziehungsweise eine Anlagerung der zu messenden Messparametern zumindest größtenteils verhindern beziehungsweise größtenteils ausschließen. Das System kann allgemein für eine Dauermessung bei kritischen Referenzwerten ausgelegt sein. Auch könnte das System bestimmte Messparameter dann messen, wenn beispielsweise ein oder mehrere Hinweise auf eine Messdurchführung bereitgestellt werden.

Weitere bevorzugte Ausgestaltungen der Erfindung ergeben sich aus den übrigen, in den Unteransprüchen genannten Merkmalen.

In weiterer bevorzugter Ausgestaltung der Erfindung ist vorgesehen, dass die zumindest eine Umweltsensoreinrichtung zumindest teilweise in einem Atemluftströmungsweg von dem Individuum und/oder zumindest teilweise in der zumindest einen Lungenfunktionsvorrichtung angeordnet ist. Somit kann ein flexibles System bereitgestellt werden, welches sich auf den jeweiligen Anwendungsfall entsprechend anpassen beziehungsweise gemäß dem Prinzip eines modularen Baukastenprinzips individuell gestalten lässt. Auch ist auf diese Weise eine individuelle Messgenauigkeit erreichbar. Je nach Anwendungsfall kann somit vor Aktivierung des Systems entschieden werden, an welcher Stelle die Umweltsensoreinrichtung vorgesehen werden soll. Für eine Dauermessung kann beispielsweise eine Verortung der Umweltsensoreinrichtung beziehungsweise zumindest von einzelnen Komponenten der Umweltsensoreinrichtung, beispielsweise einem speziellen Messsensor für einen Messparameter, direkt in den Atemluftströmungsweg vorteilhaft sein, da somit eine nahezu permanente Messreihe ermöglicht wird. Mit anderen Worten kann die Umweltsensoreinrichtung, welche in einfachster Ausführung auch nur einen einzelnen Sensor umfassen kann, direkt in einem Atemluftströmungsweg als auch unmittelbar am Gerät beziehungsweise der Lungenfunktionsvorrichtung oder der Berechnungsvorrichtung außerhalb angeordnet sein, sodass also die an der Lungenfunktionsteststelle vorhandene Luftqualität gemessen wird und ins Verhältnis zu den vom Lungentest gelieferten Ergebnissen gesetzt werden kann. So ein einzelner Sensor kann auch als Umweltsensor beschrieben oder genannt werden. Darüber hinaus ist auch eine Kombination der vorgestellten Ausführungen denkbar, sodass jeweilige Komponenten der Umweltsensoreinrichtung, beispielsweise Sensoren oder zusätzliche Funktionskomponenten, individuell entsprechend ihrer Funktion entweder innerhalb oder außerhalb parallel angeordnet werden können.

Ferner ist in einer weiteren bevorzugten Ausgestaltung der Erfindung vorgesehen, dass die zumindest eine Umweltsensoreinrichtung drahtlos oder mittels einer Übermittlungseinrichtung oder mittels eines Stecksystems mit der zumindest einen Lungenfunktionsvorrichtung verbindbar ist. Es ist somit möglich, entweder nur über eine Verbindungsart eine jeweilige Verbindung herzustellen als auch parallel verschiedene Verbindungsarten vorzuhalten. Über eine Funkverbindungseinrichtung kann somit eine besonders flexible Verbindung hergestellt werden. So eine Funkverbindungseinrichtung kann beispielsweise eine Übertragung mittels Bluetooth umfassen. Auch andere Funkverbindungen und entsprechende Komponenten für Senden und/oder Empfangen können dabei vorgesehen sein. Anteilige Komponenten der Umweltsensoreinrichtung können beispielsweise auf die Lungenfunktionsvorrichtung aufgesteckt werden, sodass eine wartungsfreundliche Ausgestaltung des Systems gewährleistet ist.

Auch ist in einer weiteren bevorzugten Ausgestaltung der Erfindung vorgesehen, dass der zumindest eine Messparameter messbare feste Teilchen von einer Atemluft des Individuums und/oder messbare gasförmige Anteile von einer Atemluft des Individuums umfasst. Feste Teilchen können beispielsweise Feinstaub, Pollen oder ganz allgemein Partikel sein, welche in der Umgebungsluft in beliebiger Konzentration vorliegen können. Dementsprechend können die gasförmigen Anteile jegliche Form von gasförmigen Stoffen sein, welche in der Umgebungsluft in beliebiger Konzentration vorliegen können. Beispielsweise können diese gasförmigen Anteile Stickstoffdioxyd, Ozon oder andere Gase sein. Auch können diese Stoffe, welche sowohl fest als auch gasförmig vorliegen können, beliebig durchmischt oder in agglomerierter Form vorliegen. Allgemein kann es sich dabei auch um luftgetragene Allergene handeln. Die festen Teilchen beziehungsweise die gasförmigen Anteile können zudem durchmischt sein mit Feuchtigkeit und in verschiedenen Temperaturen vorliegen. Die Umweltsensoreinrichtung kann entsprechend auf all diese Stoffe, sowohl fest als auch gasförmig, eingestellt beziehungsweise ausgelegt sein, diese Messparameter zu messen. Mit anderen Worten kann die Umweltsensoreinrichtung beziehungsweise entsprechende Komponenten von dieser Umweltsensoreinrichtung, etwa ein spezieller Sensor, ausgelegt sein, die zuvor genannten Messparameter sowohl bei einem Einatemvorgang als auch bei einem Ausatemvorgang zu messen.

Zudem ist in einer weiteren bevorzugten Ausgestaltung der Erfindung vorgesehen, dass die zumindest eine Information in Form einer Differenzmessung der gemessenen Messparameter in Relation zu zumindest einem Einatemvorgang und zu zumindest einem Ausatemvorgang und/oder zumindest auf ein Zeitintervall ermittelbar ist und wobei das System ausgelegt ist, einen jeweiligen Zustand des Individuums in Bezug auf einen Ruhezustand oder einen Belastungszustand des Individuums zu berücksichtigen. Beispielsweise kann die Umweltsensoreinrichtung ausgelegt sein, eine feste Anzahl von Atemzügen, zum Beispiel 100 Atemzüge oder jegliche andere Anzahl von Atemzügen, auf die jeweilige Messung zu berücksichtigen. Auch könnte die Messung über ein festes Zeitintervall, beispielsweise 10 Minuten, festgelegt werden. Zudem kann das System ausgelegt sein, einen jeweiligen Zustand des Individuums zu berücksichtigen. Zum Beispiel können die Anzahl der Atemzüge pro Zeitintervall oder jeglicher anderer Biorhythmusparameter von dem System beziehungsweise von zumindest einer Komponente des Systems gemessen werden, sodass beispielsweise ein Ruhezustand oder eine körperliche Belastung bemerkt und entsprechend bei der Berechnung von Informationen durch das System berücksichtigt werden kann.

Es ist auch in einer weiteren bevorzugten Ausgestaltung der Erfindung vorgesehen, dass das Peripheriegerät zumindest ein Mobilgerät und/oder zumindest eine Datenverarbeitungsvorrichtung ist. Das Mobilgerät kann dazu eine entsprechende Applikation aufweisen, welche mit dem System koppelbar oder sogar direkt eine weitere Komponente des Systems darstellt. Beispielsweise könnte eine solche Applikation auch eine externe zusätzliche Komponente von der Berechnungsvorrichtung sein beziehungsweise zumindest mit der Berechnungsvorrichtung kompatibel ausgelegt sein. Die Datenverarbeitungsvorrichtung kann sowohl jegliche weitere Informationen an das System übermitteln als auch als Speichereinheit für die von dem System bereitgestellten Informationen dienen. Somit ist das System besonders anwenderfreundlich und kann flexible auf verschiedenste Anwendungsfälle ausgelegt werden.

Ferner ist in einer weiteren bevorzugten Ausgestaltung der Erfindung vorgesehen, dass das Mobilgerät und/oder die zumindest eine Datenverarbeitungsvorrichtung zumindest eine Information über die Luftqualität in einer Umgebung des Individuums und/oder zumindest eine Information über den geografischen Standort des Individuums und/oder zumindest eine Information über eine Uhrzeit und/oder ein Datum bereitstellt, sodass das System diese zumindest eine jeweilige Information in Verbindung zu der Information in Form einer Differenzmessung setzen kann, sodass das System zumindest eine zusammengefasste Information erstellen und an das System bereitstellen kann.

Beispielsweise könnte die Datenverarbeitungsvorrichtung und/oder das Mobilgerät jegliche weitere Information über die Umgebungsluft aufweisen, welche außerhalb des Individuums bereits bekannt sein können. Auch können standortbezogene Daten beziehungsweise Informationen sowohl durch das Mobilgerät als auch durch die Datenverarbeitungsvorrichtung zusätzlich oder als Bestandteil der Umgebungsluftinformationen vorliegen und dem System bereitgestellt werden. Auf diese Weise können die Ergebnisse von Lungenfunktionsprüfungen (beispielsweise Spirometrie, Peak-Flow oder FeNO-Messungen und ähnliche Tests und Messungen) als auch die Differenzmessung selbst in Verbindung beziehungsweise in Korrelation zu den Umgebungsbedingungen, einschließlich der Luftqualitätsparametern mit ihren potentiellen Triggerfaktoren für Obstruktionen (Verengungen) der Atemwege (Temperatur, Feuchtigkeit, Feinstaub, Stickstoffdioxyd, Ozon, luftgetragene Allergene u.a.), gesetzt werden. Ein zusätzlicher Gewinn besteht darin, das zuvor genannte Umgebungsparameter in wechselnder Qualität, zum Beispiel innerhalb und außerhalb schadstoffbelasteter Stadtgebiete, in Verbindung zu Lungenfunktionsparametern aber auch zu Daten der gesundheitsbezogenen Lebensqualität (zum Beispiel SF 36) bei Individuen ohne oder mit obstruktiven Atemwegserkrankungen gesetzt werden können. Einem Individuum könnten zum Beispiel Informationen über die Pollenflugvorhersage oder allgemein über die datumsbezogene Belastung der Luft angezeigt werden. Auch könnten aktuelle Wetterlagen berücksichtigt werden, sodass Messungen entsprechend angepasst vollzogen werden. Auch könnten diese Daten dazu beitragen angepasste Handlungsempfehlungen anzuzeigen. Es lassen sich also mit Hilfe des Systems Informationen mittels der Datenverarbeitungsvorrichtung beziehungsweise einer entsprechenden Datenbank innerhalb dieser Datenverarbeitungsvorrichtung sammeln beziehungsweise speichern und verarbeiten, sodass ein Daten-Clustering durchgeführt werden kann. Mit den somit aufgearbeiteten Informationen lässt sich dann eine Einordnung in die Asthma-Leitlinien vollziehen und anschließend können individuelle Hinweise für die Anwender bereitgestellt werden und/oder es kann eine Speicherung in der Patientenakte mit beispielsweise einer Einordnung durch einen Arzt oder sonst einer Fachperson vollzogen werden.

Auch ist in einer weiteren bevorzugten Ausgestaltung der Erfindung vorgesehen, dass das System ausgelegt ist, eine Relation der Differenzmessung in Verbindung mit der zumindest einen Information über die Luftqualität zu zumindest einem systemischen Parameter des Individuums zu setzen, sodass das System eine weitere zusammengefasste Information erstellen und bereitstellen kann. Das System bietet somit zusätzlich die Möglichkeit die zuvor genannten Vorteile auch in Bezug auf systemische Parameter, wie zum Beispiel Herzfrequenz, Blutdruck, Atemfrequenz oder Sauerstoffsättigung, anzuwenden. Solche systemischen Parameter können somit in Verbindung beziehungsweise Korrelation gesetzt werden zu den zuvor genannten Umgebungsbedingungen einschließlich Luftqualitätsparameter mit ihren potentiellen Triggerfaktoren für Obstruktionen (Verengungen der Atemwege (Temperatur, Feuchtigkeit, Feinstaub, Stickstoffdioxyd, Ozon, luftgetragene Allergenen u.a.).

Zudem ist in einer weiteren bevorzugten Ausgestaltung der Erfindung vorgesehen, dass das System ausgelegt ist, die ermittelten Informationen in der zumindest einen Datenverarbeitungsvorrichtung zusätzlich zu sammeln, beziehungsweise zu speichern und zu verarbeiten, sodass mittels eines Daten-Clusterings eine Einordnung in zumindest ein Krankheitsbild getroffen werden kann, sodass dem Individuum mittels des zumindest einen Mobilgeräts zumindest eine Handlungsempfehlung erteilt werden kann und dieser Vorgang zudem in zumindest einer Patientenakte gespeichert werden kann. Das System kann somit einem Nutzer direkt anzeigen, welche Maßnahmen ergriffen werden können, beziehungsweise sollten, um etwa eine Steigerung des Wohlbefindens oder eine Verbesserung des Gesundheitszustands zu erreichen. Zum Beispiel könnte dies eine Anzeige auf dem Mobilgerät sein, wobei sowohl beispielsweise zumindest eine bildhafte Information als auch zumindest eine Information in Form eines Textes oder eine Mischform angezeigt werden kann. Auch ist eine parallele Form der Darstellung solcher Informationen vorstellbar. Zudem könnten die externen Informationen beispielsweise in Form von Standortdarstellungen, kartographischen Darstellungen und dergleichen parallel bereitgestellt werden. Auch könnten entsprechende Informationen derart verarbeitet werden, sodass eine Aussage über potentielle Entzündungsauslöser getroffen werden kann. Insbesondere in Bezug auf aktuelle Diskussionen über innerstädtische Luftreinhaltepläne weltweit beziehungsweise allgemein über gesundheitliche Folgen des Klimawandels vor allem unter urbanen Situationen können somit mittels des vorgestellten Systems Untersuchungsmöglichkeiten bereitgestellt werden.

Ferner ist in einer weiteren bevorzugten Ausgestaltung der Erfindung vorgesehen, dass die Messung des zumindest einen Messparameters mittels der zumindest einen Umweltsensoreinrichtung zumindest teilweise parallel oder zeitversetzt durch zumindest einen optischen und/oder akustischen Hinweis dem Individuum visualisiert darstellbar ist. Auf diese Weise ist ein besonders anwenderfreundliches System möglich.

Schlussendlich ist in einer weiteren bevorzugten Ausgestaltung der Erfindung vorgesehen, dass ein Verfahren zur Bereitstellung von zumindest einer Information über zumindest eine Wechselwirkung zwischen Umgebungsluft und Atemfunktion von einem Individuum bereitgestellt wird. Das System umfasst dabei die folgenden Schritte: Einatmen des Individuums durch zumindest eine Lungenfunktionsvorrichtung, Messen von zumindest einem Messparameter mittels zumindest einer Umweltsensoreinrichtung, Ausatmen des Individuums durch zumindest die eine Lungenfunktionsvorrichtung, Messen des gleichen Messparameters mittels der zumindest einen Umweltsensoreinrichtung, Ermitteln einer Differenzmessung der gemessenen Messparameter beim Einatemvorgang und beim Ausatemvorgang, Berechnen einer Menge des zumindest einen Messparameters, welche im Individuum nach dem Ausatemvorgang verblieben ist mittels der Umweltsensoreinrichtung und/oder einer Berechnungsvorrichtung, Bereitstellen aller gemessenen Informationen an das System, optional Senden und Empfangen von Informationen an zumindest ein externes Peripheriegerät, Bereitstellen von zumindest einer zusammengefassten Information an das System.

Die zuvor genannten Vorteile und Ausführungen für das System gelten in entsprechender Weise auch für das Verfahren. Insbesondere sind sinngemäße Erweiterungen in besonders ausgeführten Varianten des Systems auch auf das vorgestellte Verfahren übertragbar.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Anwendungsskizze von einem System zur Bereitstellung von zumindest einer Information über zumindest eine Wechselwirkung zwischen Umgebungsluft und Atemfunktion von einem Individuum;
- Figur 2: eine schematische Darstellung von einer vermessenen Lunge vor einem Einatemvorgang beziehungsweise nach einem Ausatemvorgang jeweils von Luft mit zumindest einem Messparameter;
- Figur 3: eine schematische Darstellung eines Details von einem System zur Bereitstellung von zumindest einer Information über zumindest eine Wechselwirkung zwischen Umgebungsluft und Atemfunktion von einem Individuum.

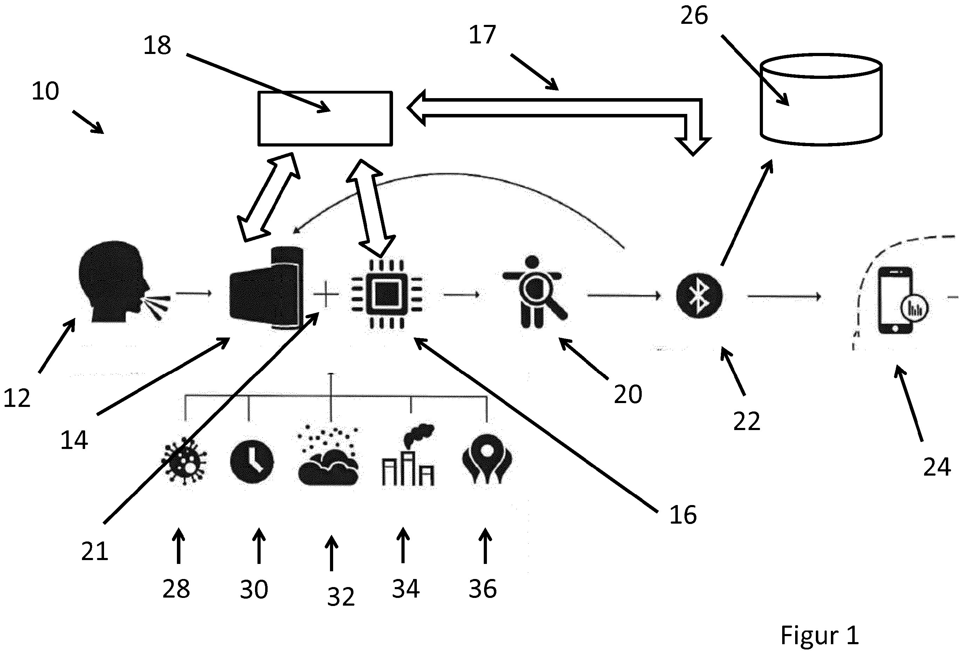

Figur 1 zeigt eine schematische Anwendungsskizze von einem System 10 zur Bereitstellung von zumindest einer Information über zumindest eine Wechselwirkung zwischen Umgebungsluft und Atemfunktion von einem Individuum 12. Das Individuum 12 respiriert in das System 10, sodass eine Lungenfunktionsvorrichtung 14 und eine Umweltsensoreinrichtung 16 entsprechend das von dem Individuum 12 respirierte Atemvolumen aufnehmen können beziehungsweise dass das Atemvolumen entsprechend an diesen beiden Komponenten vorbeigeführt wird. Eine Berechnungsvorrichtung 18 steht im funktionalen Kontakt sowohl zu der Lungenfunktionsvorrichtung 14 als auch zu der Umweltsensoreinrichtung 16. Ein Symbol 20, dargestellt als Person mit einer Lupe, zeigt an, dass eine aktive Messung entsprechend äußerlich dem Individuum 12 beziehungsweise generell einem Nutzer angezeigt werden kann. Das Individuum 12 kann beispielsweise ein Mensch sein. Das Individuum 12 kann beispielsweise ein Mensch sein, welcher zumindest ein Asthma-Phänotyp darstellt, wobei ein jeweiliger Asthma-Phänotyp unterschiedliche Auslöser für seine Symptome aufweist. Die Typisierung des Asthmas ergibt sich in der Regel zum Beispiel aus dem ärztlichen Therapieansatz. Zum Beispiel kann also dem Individuum 12, welches gleichzeitig der Nutzer des Systems sein kann, oder einem weiteren Nutzer durch ein optisches Signal oder ein akustisches Signal eine Messung angezeigt werden. Die Übermittlung kann beispielsweise elektronisch, insbesondere per email erfolgen. Zwischen der Lungenfunktionsvorrichtung 14 und der Umweltsensoreinrichtung 16 ist zudem ein Pluszeichen dargestellt, welches stellvertretend eine Übermittlungseinrichtung 21 darstellen soll. Ein Stecksystem zwischen der Lungenfunktionsvorrichtung 14 und der Umweltsensoreinrichtung 16 ist nicht gezeigt, kann aber vorgesehen werden und dann ein jegliches beliebiges Stecksystem sein. Auch eine drahtlose Verbindung zwischen der Lungenfunktionsvorrichtung 14 und der Umweltsensoreinrichtung 16 wird in dieser Figur 1 nicht gesondert dargestellt, könnte aber ebenfalls vorgesehen sein und dann eine jegliche für den Anwendungsfall praktikable Vorrichtung sein. Ein Bluetooth-Symbol 22 stellt die Verbindbarkeit des Systems 10 zu zumindest einem externen Peripheriegerät dar. Dabei zeigt ein gebogener Pfeil von rechts nach links bezogen auf die Bildebene an, dass hier eine wechselseitige Beziehung besteht. So ein externes Peripheriegerät kann zum Beispiel ein Mobilgerät 24 oder eine externe Datenverarbeitungsvorrichtung 26 sein. Eine Schnittstelle 17 zeigt an, dass die Berechnungsvorrichtung 18 über beispielsweise die Funktionalität via Bluetooth mit zumindest einem externen Peripheriegerät, beispielsweise dem Mobilgerät 24, verbindbar ist. Wie die Figur 1 darstellt, sind somit verschiedene Wege denkbar, wie die gemessenen Informationen beziehungsweise generell die Informationen gesendet, verarbeitet und ausgetauscht werden können. Unterhalb der Lungenfunktionsvorrichtung 14 und der Umweltsensoreinrichtung 16 werden verschiedene weitere Symbole dargestellt, welche einen Einfluss auf die Messung des zumindest einen Messparameters haben könnten beziehungsweise einen Messparameter selbst darstellen können. Bezogen auf die Bildebene sind zu erkennen: Ein Pollensymbol 28, ein Zeit/Datum - Symbol 30, ein Luftqualitätssymbol 32, ein Feinstaubsymbol 34 und ein Standortsymbol 36.

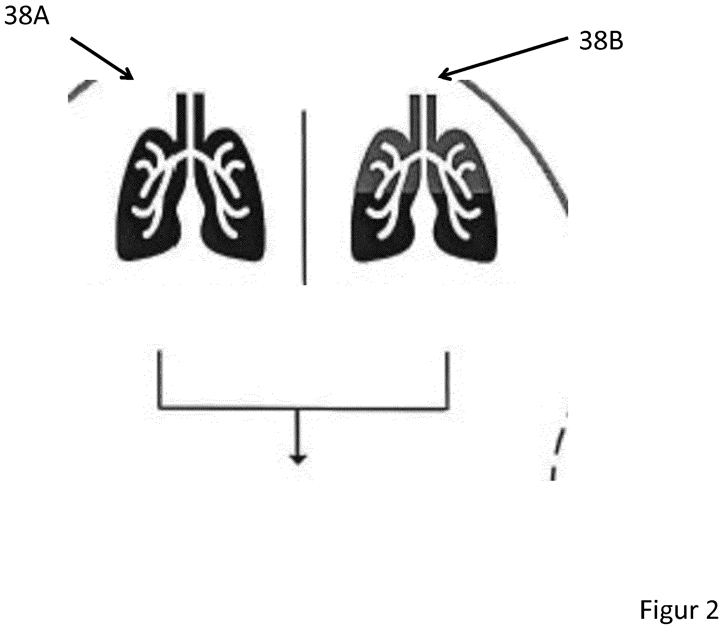

Figur 2 zeigt eine schematische Darstellung von einer zu vermessenden Lunge 38A vor einem Einatemvorgang beziehungsweise von einer zu vermessenden Lunge 38B nach einem Ausatemvorgang jeweils von Luft mit zumindest einem Messparameter, wobei die zu vermessende Lunge 38B in zwei verschiedenen Grautönen dargestellt ist. Dies soll somit symbolisch andeuten, dass zumindest eine Teilmenge des zu messenden Messparameters in der Lunge verbleibt. Ein unter den zu vermessenden Lungen 38A, B dargestellter Pfeil deutet an, dass die jeweiligen Messergebnisse beziehungsweise die jeweils bestimmten Mengen der von der Lunge absorbierten Schadstoffe beziehungsweise eines zu vermessenden Messparameters zusammengeführt werden, um eine Differenzmessung durchzuführen. Diese Informationen können sodann dem System 10 bereitgestellt werden und entsprechend weiterverarbeitet werden.

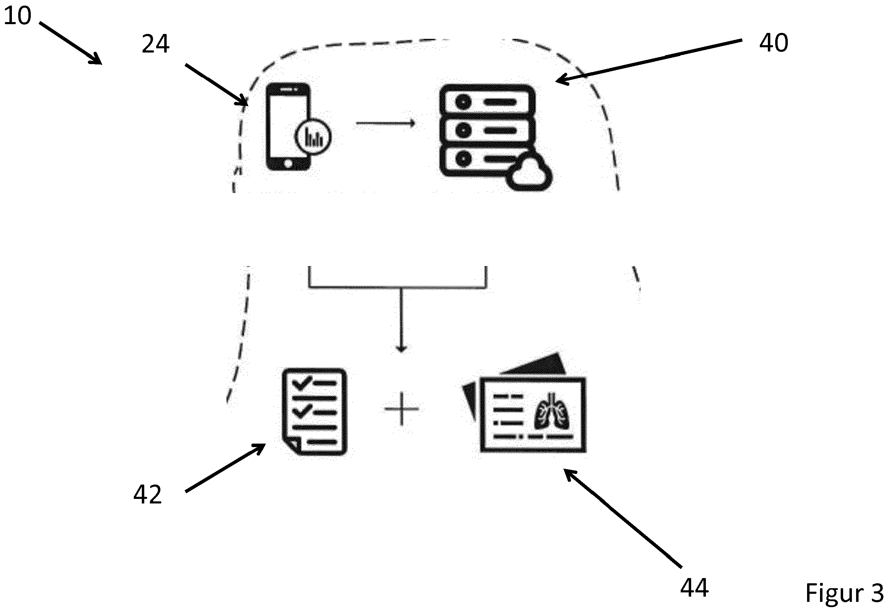

Figur 3 zeigt eine schematische Darstellung eines Details von einem System 10 zur Bereitstellung von zumindest einer Information über zumindest eine Wechselwirkung zwischen Umgebungsluft und Atemfunktion von einem Individuum 12. Zu erkennen ist ein Peripheriegerät in Form eines Mobilgeräts 24. Auf dem Mobilgerät 24 kann zum Beispiel ein Verschneiden der von Komponenten des Systems 10 empfangenen Informationen mit jeweiligen Symptomeingaben stattfinden. Die Symptomeingaben können auch von der Differenzmessung abgeleitet werden beziehungsweise können im direkten Zusammenhang mit den in der Lunge absorbierten Schadstoffen stehen. Ein Pfeil rechts neben dem Mobilgerät 24 (bezogen auf die Bildebene) deutet an, dass auf dem Mobilgerät 24 eine Verarbeitung von Informationen stattfindet. Dies wird mit einem Verarbeitungssymbol 40 dargestellt. Beispielsweise kann somit ein Verarbeiten der Informationen durchgeführt werden und somit ein Entzündungsauslöser identifiziert werden. Ein Pfeil unterhalb des dargestellten Mobilgeräts 24 und des Verarbeitungssymbols 40 deutet an, dass jegliche gemessene und extern vorliegenden Informationen beziehungsweise Eingaben von einem Nutzer beziehungsweise von dem Individuum 12 dazu dienen können, sowohl eine Handlungsanweisung als auch eine Hinterlegung in der Patientenakte durchzuführen. Dazu wird entsprechend ein Handlungsanweisungssymbol 42 und ein Patientenaktensymbol 44 dargestellt. Die Handlungsanweisungen können zum Beispiel für einen spezifischen Asthma-Phänotyp nach medizinischen Leitlinien ausgewählt sein und je nach Informationslage entsprechend ausgewählt werden.

### Bezugszeichenliste

- 10: System
- 12: Individuum
- 14: Lungenfunktionsvorrichtung
- 16: Umweltsensoreinrichtung
- 17: Schnittstelle
- 18: Berechnungsvorrichtung
- 20: Symbol
- 21: Übermittlungseinrichtung
- 22: Bluetooth-Symbol
- 24: Mobilgerät
- 26: Datenverarbeitungsvorrichtung
- 28: Pollensymbol
- 30: Zeit/Datum - Symbol
- 32: Luftqualitätssymbol
- 34: Feinstaubsymbol
- 36: Standortsymbol
- 38A: Lunge
- 38B: Lunge
- 40: Verarbeitungssymbol
- 42: Handlungsanweisungssymbol
- 44: Patientenaktensymbol

## Patentansprüche

1. System (10) zur Bereitstellung von zumindest einer Information über zumindest eine Wechselwirkung zwischen Umgebungsluft und Atemfunktion von einem Individuum (12) umfassend zumindest ein Peripheriegerät, zumindest eine Lungenfunktionsvorrichtung (14), zumindest eine mit der zumindest einen Lungenfunktionsvorrichtung (14) gekoppelten Berechnungsvorrichtung (18) umfassend zumindest eine Schnittstelle (17), sodass die Berechnungsvorrichtung (18) ausgelegt ist, mit dem zumindest einen Peripheriegerät zumindest eine Information auszutauschen, **dadurch gekennzeichnet, dass** das System (10) zudem zumindest eine mit der zumindest einen Lungenfunktionsvorrichtung (14) und/oder der zumindest einen Berechnungsvorrichtung (18) gekoppelten Umweltsensoreinrichtung (16) umfasst, welche ausgelegt ist, zumindest einen Messparameter zu messen und welche ausgelegt ist, diesen zumindest einen Messparameter sowohl bei zumindest einem Einatemvorgang des Individuums (12) und bei zumindest einem Ausatemvorgang des Individuums (12) zu messen, sodass mittels des jeweils zumindest einen gemessenen Messparameters beim Einatemvorgang und beim Ausatemvorgang zumindest die Information in Form einer Differenzmessung der gemessenen Messparameter ermittelt und an das System (10) bereitgestellt wird.

2. System (10) nach Anspruch 1, wobei die zumindest eine Umweltsensoreinrichtung (16) zumindest teilweise in einem Atemluftströmungsweg von dem Individuum (12) und/oder zumindest teilweise in der zumindest einen Lungenfunktionsvorrichtung (14) angeordnet ist.

3. System (10) nach einem der Ansprüche 1 und 2, wobei die zumindest eine Umweltsensoreinrichtung (16) drahtlos oder mittels einer Übermittlungseinrichtung (21) oder mittels eines Stecksystems mit der zumindest einen Lungenfunktionsvorrichtung (14) verbindbar ist.

4. System (10) nach einem der vorherigen Ansprüche, wobei der zumindest eine Messparameter messbare feste Teilchen von einer Atemluft des Individuums (12) und/oder messbare gasförmige Anteile von einer Atemluft des Individuums (12) umfasst.

5. System (10) nach einem der vorherigen Ansprüche, wobei die zumindest eine Information in Form einer Differenzmessung der gemessenen Messparameter in Relation zu zumindest einem Einatemvorgang und zumindest zu einem Ausatemvorgang und/oder zumindest auf ein Zeitintervall ermittelbar ist und wobei das System (10) ausgelegt ist, einen jeweiligen Zustand des Individuums (12) in Bezug auf einen Ruhezustand oder einen Belastungszustand des Individuums (12) zu berücksichtigen.

6. System (10) nach einem der vorherigen Ansprüche, wobei das Peripheriegerät zumindest ein Mobilgerät (24) und/oder zumindest eine Datenverarbeitungsvorrichtung (26) ist.

7. System (10) nach Anspruch 6, wobei das Mobilgerät (24) und/oder die zumindest eine Datenverarbeitungsvorrichtung (26) zumindest eine Information über die Luftqualität in einer Umgebung des Individuums (12) und/oder zumindest eine Information über den geografischen Standort des Individuums (12) und/oder zumindest eine Information über eine Uhrzeit und/oder ein Datum bereitstellt, sodass das System (10) diese zumindest eine jeweilige Information in Verbindung zu der Information in Form einer Differenzmessung setzen kann, sodass das System (10) zumindest eine zusammengefasste Information erstellen und an das System (10) bereitstellen kann.

8. System (10) nach Anspruch 7, wobei das System (10) ausgelegt ist eine Relation der Differenzmessung in Verbindung mit der zumindest einen Information über die Luftqualität zu zumindest einem systemischen Parameter des Individuums (12) zu setzen, sodass das System (10) eine weitere zusammengefasste Information erstellen und bereitstellen kann.

9. System (10) nach einem der vorherigen Ansprüche 6 bis 8, wobei das System (10) ausgelegt ist die ermittelten Informationen in der zumindest einen Datenverarbeitungsvorrichtung (26) zusätzlich zu speichern und zu verarbeiten, sodass mittels eines Daten-Clusterings eine Einordnung in zumindest ein Krankheitsbild getroffen werden kann, sodass dem Individuum (12) mittels des zumindest einen Mobilgeräts (24) zumindest eine Handlungsempfehlung erteilt werden kann und dieser Vorgang zudem in zumindest einer Patientenakte gespeichert werden kann.

10. System (10) nach einem der vorherigen Ansprüche, wobei die Messung des zumindest einen Messparameters mittels der zumindest einen Umweltsensoreinrichtung (16) zumindest teilweise parallel oder zeitversetzt durch zumindest einen optischen und/oder akustischen Hinweis dem Individuum (12) mitgeteilt wird.

11. Verfahren zur Bereitstellung von zumindest einer Information über zumindest eine Wechselwirkung zwischen Umgebungsluft und Atemfunktion von einem Individuum (12) umfassend die folgenden Schritte:
• Einatmen des Individuums (12) durch zumindest eine Lungenfunktionsvorrichtung (14);
• Messen von zumindest einem Messparameter mittels zumindest einer Umweltsensoreinrichtung (16);
• Ausatmen des Individuums (12) durch zumindest die eine Lungenfunktionsvorrichtung (14);
• Messen des gleichen Messparameters mittels der zumindest einen Umweltsensoreinrichtung (16);
• Ermitteln einer Differenzmessung der gemessenen Messparameter beim Einatemvorgang und beim Ausatemvorgang;
• Berechnen einer Menge des zumindest einen Messparameters, welche im Individuum (12) nach dem Ausatemvorgang verblieben ist mittels der Umweltsensoreinrichtung (16) und/oder einer Berechnungsvorrichtung (18);
• Bereitstellen aller gemessenen Informationen an das System (10);
• Optional Senden und Empfangen von Informationen an zumindest ein externes Peripheriegerät;
• Bereitstellen von zumindest einer zusammengefassten Information an das System (10).

## Claims

1. A System (10) for providing at least one information about at least one interaction between environmental air and respiratory function of an individual (12), comprising at least one peripheral device, at least one lung function device (14), at least one calculation device (18) coupled to said at least one lung function device (14) and comprising at least one interface (17), such that the calculation device (18) is adapted to exchange at least one information with the at least one peripheral device, **characterised in that** the system (10) further comprises at least one environmental sensor device (16) coupled to said at least one lung function device (14) and/or said at least one calculation device (18) which is adapted to measure at least one measurement parameter and adapted to measure this at least one measurement parameter both during at least one inhalation process of the individual (12) and during at least one exhalation process of the individual (12), so that, by means of the at least one measurement parameter measured in each case during the inhalation process and during the exhalation process, at least the information in the form of a differential measurement of the measured measurement parameters is determined and provided to the system (10).

2. System (10) according to claim 1, wherein said at least one environmental sensor means (16) is at least partially disposed in a respiratory airflow path from said individual (12) and/or at least partially disposed in said at least one lung function device (14).

3. System (10) according to any one of claims 1 and 2, wherein the at least one environmental sensor device (16) is connectable to the at least one lung function device (14) wirelessly or by means of a transmission device (21) or by means of a plug-in system.

4. System (10) according to any one of the preceding claims, wherein the at least one measurement parameter comprises measurable solid particles from a breath of the individual (12) and/or measurable gaseous components from a breath of the individual (12).

5. System (10) according to any one of the previous claims, wherein the at least one information can be determined in the form of a differential measurement of the measured measurement parameters in relation to at least one inhalation process and at least one exhalation process and/or at least to a time interval, and wherein the system (10) is designed to take into account a respective state of the individual (12) with respect to a resting state or a stress state of the individual (12).

6. System (10) according to any one of the preceding claims, wherein the peripheral device is at least one mobile device (24) and/or at least one data processing device (26).

7. System (10) according to claim 6, wherein the mobile device (24) and/or the at least one data processing device (26) provides at least one information about the air quality in an environment of the individual (12) and/or at least one information about the geographical location of the individual (12) and/or at least one information about a time and/or a date, so that the system (10) can relate this at least one respective information to the information in the form of a difference measurement, so that the system (10) can create at least one summarized information and provide it to the system (10).

8. System (10) according to claim 7, wherein the system (10) is adapted to relate the differential measurement in conjunction with the at least one air quality information to at least one systemic parameter of the individual (12) such that the system (10) can generate and provide a further summarised information.

9. System (10) according to any one of the preceding claims 6 to 8, wherein the system (10) is designed to additionally store and process the determined information in the at least one data processing device (26), so that a classification into at least one clinical picture can be made by means of a data clustering, so that the individual (12) can be given at least one recommendation for action by means of the at least one mobile device (24) and this process can additionally be stored in at least one patient file.

10. System (10) according to one of the previous claims, wherein the measurement of the at least one measurement parameter by means of the at least one environmental sensor device (16) is communicated to the individual (12) at least partially in parallel or with a time delay by at least one visual and/or acoustic indication.

11. Method of providing at least one information about at least one interaction between ambient air and respiratory function of an individual (12) comprising the steps of:
• inhalation of the individual (12) by means of at least one lung function device (14);
• measuring at least one measurement parameter by means of at least one environmental sensor device (16);
• exhalation of the individual (12) through at least the one lung function device (14);
• measuring the same measurement parameter by means of the at least one environmental sensor device (16);
• determining a difference measurement of the measured measurement parameters during inhalation and exhalation;
• calculating an amount of the at least one measurement parameter remaining in the individual (12) after the exhalation process by means of the environmental sensor device (16) and/or a calculation device (18);
• providing all measured information to the system (10);
• optionally sending and receiving information to at least one external peripheral device;
• providing at least one summary information to the system (10).

## Revendications

1. Système (10) pour fournir au moins une information sur au moins une interaction entre l'air ambiant et la fonction respiratoire d'un individu (12) comprenant au moins un appareil périphérique, au moins un dispositif de fonction pulmonaire (14), au moins un dispositif de calcul (18) couplé audit au moins un dispositif de fonction pulmonaire (14) comprenant au moins une interface (17), de sorte que le dispositif de calcul (18) est adapté pour échanger au moins une information avec l'au moins un appareil périphérique, **caractérisé en ce que** le système (10) comprend en outre au moins un dispositif de capteur d'environnement (16) couplé à l'au moins un dispositif de fonction pulmonaire (14) et/ou à l'au moins un dispositif de calcul (18), qui est conçu pour mesurer au moins un paramètre de mesure et qui est conçu pour mesurer cet au moins un paramètre de mesure aussi bien lors d'au moins un processus d'inspiration de l'individu (12) que lors d'au moins un processus d'expiration de l'individu (12), de sorte qu'au moyen de l'au moins un paramètre de mesure mesuré respectivement lors du processus d'inspiration et lors du processus d'expiration, au moins l'information est déterminée sous la forme d'une mesure de différence des paramètres de mesure mesurés et est mise à disposition du système (10).

2. Système (10) selon la revendication 1, dans lequel l'au moins un dispositif de capteur d'environnement (16) est disposé au moins partiellement dans un trajet d'écoulement d'air respiratoire provenant dudit individu (12) et/ou au moins partiellement dans l'au moins un dispositif de fonction pulmonaire (14).

3. Système (10) selon l'une des revendications 1 et 2, dans lequel l'au moins un dispositif capteur d'environnement (16) peut être relié sans fil ou au moyen d'un dispositif de transmission (21) ou au moyen d'un système de connexion audit au moins un dispositif de fonction pulmonaire (14).

4. Système (10) selon l'une quelconque des revendications précédentes, dans lequel l'au moins un paramètre de mesure comprend des particules solides mesurables d'un air respiré par l'individu (12) et/ou des fractions gazeuses mesurables d'un air respiré par l'individu (12).

5. Système (10) selon l'une des revendications précédentes, dans lequel l'au moins une information peut être déterminée sous la forme d'une mesure différentielle des paramètres de mesure mesurés par rapport à au moins un processus d'inspiration et au moins un processus d'expiration et/ou au moins sur un intervalle de temps, et dans lequel le système (10) est conçu pour prendre en compte un état respectif de l'individu (12) par rapport à un état de repos ou un état d'effort de l'individu (12).

6. Système (10) selon l'une des revendications précédentes, dans lequel le périphérique est au moins un appareil mobile (24) et/ou au moins un dispositif de traitement de données (26).

7. Système (10) selon la revendication 6, dans lequel l'appareil mobile (24) et/ou le au moins un dispositif de traitement de données (26) fournit au moins une information sur la qualité de l'air dans un environnement de l'individu (12) et/ou au moins une information sur la localisation géographique de l'individu (12) et/ou au moins une information sur une heure et/ou une date, de sorte que le système (10) peut mettre en relation cette au moins une information respective avec l'information sous la forme d'une mesure de différence, de sorte que le système (10) peut créer et fournir au moins une information résumée au système (10).

8. Système (10) selon la revendication 7, dans lequel le système (10) est adapté pour établir une relation entre la mesure différentielle en relation avec l'au moins une information sur la qualité de l'air et au moins un paramètre systémique de l'individu (12), de sorte que le système (10) peut créer et fournir une autre information résumée.

9. Système (10) selon l'une des revendications précédentes 6 à 8, le système (10) étant conçu pour stocker et traiter en outre les informations déterminées dans l'au moins un dispositif de traitement de données (26), de sorte qu'une classification dans au moins un tableau clinique peut être effectuée au moyen d'un regroupement de données, de sorte qu'au moins une recommandation d'action peut être donnée à l'individu (12) au moyen de l'au moins un appareil mobile (24) et que ce processus peut en outre être stocké dans au moins un dossier de patient.

10. Système (10) selon l'une des revendications précédentes, dans lequel la mesure de l'au moins un paramètre de mesure au moyen de l'au moins un dispositif de capteur d'environnement (16) est communiquée au moins partiellement en parallèle ou en différé à l'individu (12) par au moins une indication optique et/ou acoustique.

11. Procédé pour fournir au moins une information sur au moins une interaction entre l'air ambiant et la fonction respiratoire d'un individu (12), comprenant les étapes suivantes :
• l'inhalation de l'individu (12) au moyen de l'au moins un dispositif de fonction pulmonaire (14) ;
• la mesure d'au moins un paramètre de mesure au moyen d'au moins un dispositif capteur d'environnement (16) ;
• l'expiration de l'individu (12) au moyen de l'au moins un dispositif de fonction pulmonaire (14) ;
• la mesure du même paramètre de mesure au moyen de l'moins un dispositif capteur d'environnement (16) ;
• la détermination d'une mesure différentielle des paramètres de mesure mesurés lors du processus d'inspiration et du processus d'expiration ;
• le calcul d'une quantité dudit au moins un paramètre de mesure qui est restée dans l'individu (12) après le processus d'expiration au moyen du dispositif de capteur d'environnement (16) et/ou d'un dispositif de calcul (18) ;
• la mise à disposition de toutes les informations mesurées au système (10) ;
• optionellement, l'envoi et la réception d'informations à au moins un périphérique externe
• la fourniture d'au moins une information résumée au système (10).
